# EUROPEAN PATENT SPECIFICATION

(11) **EP 1 890 707 B1**
(45) Date of publication and mention of the grant of the patent: **14.09.2011**
(21) Application number: 05816351.0
(22) Date of filing: 16.12.2005
(51) Int. Cl.: A61K 31/70, A61P 31/18, A23L 1/29

(54) **NUTRITIONAL SUPPLEMENT WITH COLOSTRUM AND EPA AND OPTIONALLY DHA OR GLA**
NAHRUNGSERGÄNZUNG MIT COLOSTRUM UND EPA UND GEGEBENFALLS DHA ODER GLA
SUPPLÉMENT NUTRITIONNEL AVEC COLOSTRUM ET EPA ET EVENTUELLEMENT DHA OU GLA

(30) Priority: 21.04.2005 EP 05103257
(43) Date of publication of application: 27.02.2008
(73) Proprietor: N.V. Nutricia, 2712 HM Zoetermeer (NL)
(72) Inventor: VAN DEN BERG, Jeroen, Johannes, Maria, Aldgate SA 5154 (AU); VAN TOL, Eric, Alexander, Franciscus, NL-6824 MZ Arnhem (NL); SIJBEN, Johanne, Wilhelmus, Christina, 6707 AN Wageningen (NL); HOIJER, Maarten Anne, NL-6814 JA Arnhem (NL)
(74) Representative: Swinkels, Bart Willem
(86) International application number: PCT/NL2005/050082
(87) International publication number: WO 2006/112694

(56) References cited:
- EP-A2- 0 711 503
- WO-A-98/11910
- WO-A-2004/112508
- US-A1- 2002 016 289
- US-A1- 2004 122 105
- US-B1- 6 974 841
- FIDLER N ET AL: "Polyunsaturated fatty acid composition of human colostrum lipids in Slovenia: regional differences." FOOD TECHNOLOGY AND BIOTECHNOLOGY 38 (2) 149-153 2000 BIOTECH. FAC., INST. OF NUTR., UNIV. OF LJUBLJANA, SI-1230 DOMZALE, SLOVENIA. TEL. ++386 (0)1 268 642. FAX ++386 (0)1 721 005. E-MAIL NATASA.FIDLER(A)GUEST.ARNES.SI, 2000, XP008071463
- BOERSMA E R ET AL: "Vitamin E, lipid fractions, and fatty acid composition of colostrum, transitional milk, and mature milk: an international comparative study." AMERICAN JOURNAL OF CLINICAL NUTRITION 1991 DEP. OF OBSTETRICS & GYNAECOLOGY, STATE UNIV. GRONINGEN, EZ GRONINGEN 9713, NETHERLANDS, vol. 53, no. 5, 1991, page 1197, XP008071461
- FIDLER NATASA ET AL: "The fatty acid composition of human colostrum" EUROPEAN JOURNAL OF NUTRITION, vol. 39, no. 1, February 2000 (2000-02), pages 31-37, XP008071462 ISSN: 1436-6207

## Description

### FIELD OF THE INVENTION

The invention relates to a nutritional composition. In particular the invention relates to the use of a nutritional composition for the treatment of intestinal dysfunction in HIV patients.

### BACKGROUND OF THE INVENTION

Intestinal dysfunction in HIV patients is the resultant of both viral infection and inflammatory reactivity. Intestinal dysfunction is often related to inflammatory conditions in the gut. Increased intestinal permeability or inadequate intestinal barrier function are pathological features often seen in patients with chronic inflammatory diseases, allergies, food poisoning, and HIV infection.

Nutritional solutions have been proposed in the past. WO2004/112509 describes a nutritional formula for optimal gut barrier maturation in newborn infants. The proposed formula contains at least one microorganism, EPA and non-digestible oligosaccharides.

The present inventors described in a previous application (PCT/NL2004/000444) that polyunsaturated fatty acids, particularly eicosapentaenoic acid (EPA), docosahexaenoic acid (DHA) and arachidonic acid (ARA), are capable of effectively improving intestinal barrier resistance and reducing intestinal tight junction permeability. It was further described that the precursor of ARA, gamma linolenic acid (GLA), could also be used without negatively influencing the effectiveness.

### SUMMARY OF THE INVENTION

The present invention provides a combination of selected polyunsaturated fatty acids (PUFA) and colostrum from cows. The combination of PUFA and colostrum effectively improves gut function by improving barrier integrity and supporting tissue regeneration. Intestinal barrier integrity is improved by synergistically reducing the intestinal permeability and improving mucus production. The latter is of particular importance for improving barrier integrity in HIV patients challenged with inflammatory conditions in the gut and more specifically adult HIV patients.

Surprisingly the present inventors found that colostrum synergistically acts with the PUFA to decrease the intestinal permeability and to support tissue regeneration, thereby improving gut function.

In one embodiment the invention therefore concerns a composition comprising colostrum from cows and a fat blend comprising EPA, whereby EPA is present in the range of 10-40 wt% of the total fat blend. In a further embodiment the fat blend in the present composition also comprises DHA, preferably the fat blend comprises between 1-25 wt% DHA of the total fat blend. In another embodiment the fat blend in the present composition further comprises GLA, preferably the fat blend further comprises 1-15 wt% GLA of the total fat blend. In yet another embodiment the fat blend in the present composition further comprises DHA and GLA, preferably in the specified amounts as defined above.

One aspect of the invention is the use of colostrum from cows and a fat blend comprising 10-40% EPA, for the manufacture of a nutritional composition for prevention and/or the treatment of intestinal dysfunction in HIV patients

### DETAILED DESCRIPTION OF THE INVENTION

It is estimated that up to 25-30% of HIV infected patients without substantial clinical symptoms have an abnormal gut function such as decreased uptake of nutrients and/or increased permeability due to leakage of the epithelial lining of the gut. The prevalence of these aspects of gut dysfunction are known to increase during the development of disease. Indeed, it is thought that eventually almost all AIDS patients have compromised gut functions to some extent. The composition of the present invention can advantageously be used to improve the intestinal function of HIV patients.

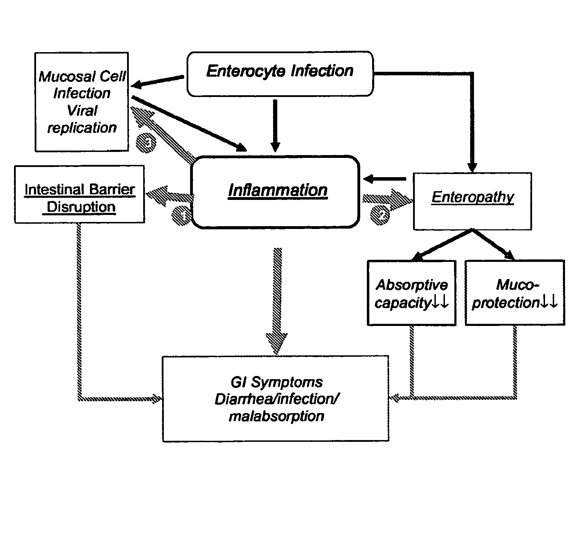

Without being bound by theory, in fig 1 a scheme is proposed wherein the factors involved are depicted that during HIV infection lead to an abnormal gut function ultimately characterized by clinical manifestations such as diarrhea, infection and malabsorption.

The infection of the enterocyte with the HIV is proposed to result in an inflammatory reaction that ultimately could lead to gastro-intestinal (GI) symptoms that are often seen in HIV patient such as diarrhea, other infections and malabsorption. This reaction can be directly mediated by the inflammatory reaction or indirectly the intestinal barrier disruption or enteropathy (decreased absorption and mucus production). The ingredients proposed all have their effects in one or more of the arrows in the scheme.

### Colostrum

Colostrum is the pre-milk liquid secreted by the mammary glands of mammalian mothers after giving birth, in particular cows after calving. Colostrum contains many biologically active ingredients and is therefore an excellent source of biologically active molecules such as growth factors and immunoglobulins. Colostrum may be in a liquid form or a dry form. In the context of the present invention suitably the colostrum is in the form of a concentrated protein powder that can be prepared as described in e.g. US6202546. Commercial colostrum powder comprises about 70-80 wt% protein. Liquid colostrum comprises between 4-20 wt% protein.
Colostrum protein herein refers to the protein fraction present in colostrum either in liquid or in dry form such as in the form of a concentrated protein powder. Preferably the protein is essentially undenatured such that the biologically active molecules that are present in the protein fraction are not inactivated.

For having beneficial effects in HIV patients between about 5 and 50 gram colostrum protein is provided on a daily basis, preferably between 10 and 30 gram, most preferably about 15 g colostrum protein per day. As already mentioned, commercial colostrum powder comprises about 70-80 wt% protein. Therefore the products according to the invention comprise between about 6 and 75 g colostrum powder in a daily dose. It is preferred to provide sufficient colostrum such that at least 1 gram immunoglobulin G, preferably between 1-10 and most preferred between 2 - 8 gram immunoglobulin G per day is provided.

Extracts from colostrum or milk, such as a whey growth factor extract as described in EP0545946 or a casein extract as described in WO02083164, immunoglobulin concentrates, lactoferrin or other concentrated whey fractions can also be used to improve the intestinal barrier function of HIV patients. Without being bound by theory it is thought that the growth factors and/or antibodies are involved in the improved regeneration of intestinal tissue and thereby improve the gut integrity. Preferably the colostrum or whey extracts comprise at least the same or higher amounts of immunoglobulins and growth factors as present in fresh colostrum obtained from cows in the period 1-5 days after calving, preferably 1-3 days after calving. Based on dry matter, the amount of immunoglobulin-G in the colostrum preferably is between 20 and 40 wt%. The growth factors present in colostrum comprise but are not limited to, Insulin like growth factor 1 and 2, Transforming growth factor 1 and 2, betacellulin, KGF and others known to the skilled person. Preferably the concentrations of these growth factors are at least in the same range (based on dry weight) as in fresh liquid colostrums, which are commonly known or can be measured. The advantage of using extracts is that without increasing the amount of protein too much, the immunoglobulin and growth factor content of the product according to the invention can be increased.

### Polyunsaturated fatty acids:

In the context of this invention the term fat blend refers to a composition comprising at least EPA with at least one other fatty acid belonging to the group of n-3 fatty acids and/or n-6 fatty acids.

As already mentioned polyunsaturated fatty acids, particularly eicosapentaenoic acid (EPA, C20:5n-3), docosahexaenoic acid (DHA) and arachidonic acid (ARA), are capable of effectively reducing intestinal tight junction permeability. The precursor of ARA, gamma linolenic acid (GLA, C18:3n-6), can be used without having a negative effect on the effectiveness. This is advantageous because GLA is less inflammatory than ARA. Therefore fat blends comprising EPA and optionally DHA and/or with GLA are preferred. The above-mentioned fatty acids effectively support epithelial resistance and can reduce increased epithelial permeability caused by inflammatory conditions. In addition, targeting the mucosal inflammatory reactivity may reduce the detrimental effect of the inflammatory mediators on intestinal barrier function and epithelial absorptive capacity. Hence a composition, suitable for improving intestinal barrier integrity is provided, which, besides colostrum, comprises EPA and preferably GLA and DHA.

Based on the biochemical pathways it can be hypothesized that other combinations of fatty acids are also effective. Thus, compositions comprising one or more other PUFA or mixtures (fat blend) thereof are also provided. For example a fat blend comprising a mixture of EPA, and any of docosahexaenoic acid (DHA, C22:6n-3), dihomo-gamma linolenic acid (DGLA, C20:3n-6), stearidonic acid (STA, C18:4n-3), alpha linolenic acid (ALA, C18:3n-3), (docosapentaenoic acid (DPA, C22:5n-3), eicosatetraenoic acid (ETE, C20:4n-3) and/or arachidonic acid (ARA, n-6) may be used. In particular a fat blend comprising a mixure of EPA and any of DHA, DGLA, STA, ALA, DPA, ETE and/or ARA may be used.

In one embodiment at least about 25 en%, preferably at least about 30 en%, more preferably at least about 35 en% of a fat blend comprising n-3 and/or n-6 fatty acids is used (en% is short for energy percentage and represents the relative amount each constituent contributes to the total caloric value of the preparation). Suitably a relatively high daily dose of the polyunsaturated fatty acids is used. Preferred daily amounts are at least 1 gram PUFA, preferably between 1-25 gram PUFA, more preferably between 2 and 15 gram PUFA and most preferred is an amount between 3 and 10 gram PUFA.

An optimal fat blend preferably comprises an n-3/n-6 fatty acid ratio between 1-3. A ratio between 1.5 and 2.7 is even more preferred because this will give optimal stability of the product comprising the fat blend. Further, the weight percentage in the fat blend of n-3 is between 15 and 50, and is most preferably between 20-50 wt% of total fatty acid content, and the weight percentage of n-6 is between 10-50, preferably between 10-40 wt% and is most preferably between 15-35 wt% of total fatty acid content of the fat blend. The other fatty acids present in the complete product should not significantly affect the n-3/n-6 fatty acid ratio. The ratio n-3/n-6 of the complete product should stay between 1-3 and preferably between 1.5 and 2.7. Suitably an optimal fat blend therefore may comprise between 40 wt% and 60 wt% borage oil and between 40% and 60% fish oil.

Preferred daily amounts, or in other words daily doses, are used of at least 0.15 gram EPA. Preferably between 0.15 and 5 gram EPA. In those embodiments of the invention where DHA and/or GLA are included between 0.10 and 4.0 gram DHA and between 0.05 and 2.5 gram GLA are used. Preferably, especially in those cases where besides EPA DHA and/or GLA are used, between 0.5 and 2.5 gram EPA, between 0.3 and 2.0 gram DHA and between 0.25 and 1.25 gram GLA are used. Most preferred are amounts between 0.75 and 1.5 gram EPA, between 0.5 and 1.2 gram DHA and between 0.37 and 1.0 gram GLA. In one embodiment the nutritional composition comprises between 0.15 and 5 g EPA and between 0.05 and 2.5 g GLA in a daily dose.

In order not to compromise the stability and the taste of the product, which can be of relevance for good compliance, it is important that the ratio of the fatty acids and the colostrum is within certain ranges. The product preferably comprises per gram colostrum protein between 0.01 - 0.30 gram EPA, and if present between 0.006 - 0.25 gram DHA and between 0.003 - 0.17 gram GLA per gram colostrum protein. A The composition of the comprises colostrum from cows and a fat blend with EPA and EPA is present in the range of 10-40 wt% EPA of the total fat blend. More preferably the composition further comprises between 1-25 wt% DHA of the total fat blend or the fat blend further comprises between 1-15 wt% GLA of the total fat blend. More preferably the fat blend further comprises between 1-25 wt% DHA and between 1-15 wt% GLA of the total fat blend

### Cysteine or source of cysteine

Surprisingly, the present inventors also found that extra cysteine in the form of NAC or protein material rich in cysteine, is capable to further improve the intestinal barrier function in HIV patients with intestinal inflammation. Therefore in another embodiment the invention provides the addition of a cysteine source to the nutritional composition that can further improve the gut barrier integrity.

The compositions provided, optionally further comprise in addition to one or more ingredients as described above, a suitable amount of cysteine and/or source of cysteine. The phrase "source of cysteine" herein refers to all compounds that contain a biologically available cysteine, in any form, and is calculated as the amount of cysteine amino acid that is present in a compound, or can be derived from a compound in the body after ingestion, on a molar basis. During intestinal inflammation the inventors found that the intestinal function can be improved by the addition of cysteine, a source of cysteine or both to the nutritional composition comprising fat blend and colostrum

Herein below "cysteine equivalent" refers to an amount of cysteine as such or to an amount of cysteine that is present in a source of cysteine. For example 100 mg NAC (N-acetyl cysteine; MW= 163.2) is equivalent to 74 mg cysteine (MW 121.15). Similarly this can be applied to proteins or peptides. When a peptide (MW = X Dalton) contains 3 cysteine amino acids (3YDalton), than 100 mg of this peptide is equivalent to 100x3Y/X mg cysteine. Thus 100mg of this peptide is 300Y/X mg cysteine equivalent.

Suitable sources of cysteine according to the invention are, for example, proteins in denatured and/or undenatured form such as milk proteins e.g. whey proteins such as alpha-lactalbumin or albumin and β-lactoglobulin, or egg proteins. These proteins are rich in cysteine and are therefore particularly suitable. Plant proteins such as pea, potato, soy and rice can also be used to provide cysteine. Also hydrolysates of these protein sources can be used or fractions enriched for cysteine rich proteins or peptides (e.g. as described in EP 1201137). Furthermore, synthetic cysteine equivalents, e.g. derivatives of cysteine, such as glutathione, cysteine, cysteine salts, N-acetyl cysteine and/or diacetyl cysteine can be used. In one embodiment the present composition comprises cysteine wherein the source of cysteine is selected from the group consisting of N-Acetyl cysteine, whey, egg proteins or a combination thereof.

The HIV infected target patients are suitably administered a daily dose of at least about 100 mg cysteine equivalent, preferably at least about 200, 400, or 600 mg cysteine equivalent per day, more preferably at least about 1000 mg cysteine equivalent per day. It is understood that a daily dosage can be subdivided into 2, 3 or more dosage units taken several times a day.

In yet another embodiment the compositions according to the invention optionally further comprises one or more compounds that can enhance systemic and/or tissue levels of glutathione. Among these, lipoic acid, pyruvate, oxaloacetate, oxaloaspartate, all have been found to stimulate glutathione levels. Such glutathione level stimulating compounds may be used in addition to cysteine but also instead of cysteine.

### Nutritional compositions

The nutritional compositions of the current invention are particularly beneficial for patients with HIV. The present nutritional compositions can have a varying energy density, but because HIV patients with intestinal dysfunction are not likely to have much appetite it is beneficial to provide the daily dose of all the ingredients in a small volume. One preferred embodiment therefore is a nutritional bar wherein all the ingredients are packaged within a small volume with a high density. Furthermore, another advantage of processing the ingredients in the form of a bar is improvement of taste and stability of the composition. In case the product is made in the form of a bar, preferably one bar of maximal 100 g is administered per day in a single dose, but also multiple doses of smaller bars are possible preferably not exceeding the maximum of about 100 g per day.

Another preferred embodiment is a liquid composition wherein the ingredients are supplied in such a way that only a small volume is necessary and the taste and stability of the ingredients is guaranteed. Preferably not more then 250 ml of the liquid composition or even more preferably not more than 150 ml per daily dose is used to administer the composition according to the present invention.

The advantage of such a compact nutritional product is that when HIV patient lack sufficient appetite, the small volume will improve the compliance and loyalty to the product. When the lack of appetite is severe, the product could also be administered through tube feeding regimens e.g. as nighttime feeding. The viscosity of tube feed should be in the range of 1 - 100, preferably 1.2 - 30 and most preferred between 1.5 - 20 N.s/m² at a shear rate of 100 s⁻¹ at 20°C.

A complete nutritional formula can be particularly beneficial for HIV patients lacking sufficient nutritional support and require additional macronutrients such as proteins, fat and carbohydrates as well as micronutrients such as vitamins and minerals. A preferred embodiment therefore comprises nutritionally high value proteins, fats, carbohydrates, minerals and vitamins wherein the vitamins and minerals need to be present in adequate amounts as required by Food for Special Medical Purposes (FSMP) regulations. A preferred embodiment of the present composition comprises between 15 and 50 en% lipid, between 25 and 60 en% colostrum protein, between 15 and 45 en% carbohydrate and cysteine or source of cysteine selected from the group consisting of NAC, whey, colostrum, egg proteins or combinations thereof.

Another aspect of the invention is a nutritional regimen e.g. a combination of solid (dry) products and liquid products to further improve the pleasantness e.g. the taste and compliance of the products. The nutritional regimen comprises the administration of a bar that contains amongst other things colostrum and preferably NAC and the administration of a liquid product comprising the ingredients that can withstand liquid processing steps, such as sterilization and pasteurization, like the fat blend according to the invention, fibers, etc. Hence in one aspect the invention concerns a nutritional kit of parts comprising at least one solid composition a) and at least one liquid composition b), wherein solid composition a) comprises colostrum from cows and preferably NAC, and composition b) comprises a fat blend comprising 10-40 wt% EPA, 1-25 wt% DHA and 1-15 wt% GLA.

Diarrhea is a major problem in many HIV patients that receive liquid foods. It was found that stool problems are reduced by administering the present colostrum composition as a dry nutritional composition or as a liquid nutritional composition that has an osmolality between 50 and 500 mOsm/kg, more preferably between 100 and 400 mOsm/kg.

In view of the above, the nutritional composition preferably should not deliver excessive amounts of calories. Hence, the nutritional composition preferably contains not more that 500 kcal/daily dose, more preferably between 200 and 400 kcal/daily dose and more preferably between 250 and 350 kcal/daily dose.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

### EXAMPLES

### Example 1. Liquid nutritional composition for the treatment of intestinal dysfunction in HIV patients

| **Raw Material** | **g/ 100ml** |
|---|---|
| borage oil | 2.0 |
| EPA-DHA oil | 3.0 |
| Colostrum | 10.0 (7.5g protein) |
| MaltoDextrin | 20.0 |
| | **The daily dose is 100-300 ml, preferably 200 ml.** |

### Example 2. Powder nutritional composition for HIV patients

| **Raw Material** | **g/ day** | **g/ 100g** |
|---|---|---|
| Colostrum | 20.00 | 26.27 |
| NAC | 1.2 | 1.55 |
| borage oil | 4.00 | 5.25 |
| EPA-DHA oil | 6.00 | 7.88 |
| alpha-lactalbumin | 34.03 | 44.69 |
| MaltoDex DE47 | 7.00 | 9.19 |
| MaltoDex | 5.00 | 6.57 |
| SSL (emulsifier) | 0.11 | 0.15 |
| Vit/mineral mixture | according to FSMP regulations | |

| | **per day kcal** | **En%** | **per 100g kcal** |
|---|---|---|---|
| energy protein | 185 | 56.0 | 185 |
| energy carbohydrates | 56 | 17.0 | 86 |
| energy fat | 89 | 27.0 | 100 |
| *SUM* | *330* | 100 | *371* |

### Example 3. Nutritional regimen of liquid and solid products for improved compliance.

A nutritional kit of parts comprising 100 ml of a liquid nutritional composition comprising: 2.0 g borage oil and 3 g EPA/DHA oil, and 100g of a solid or non-aqueous composition with a water activity of less than 0.7 at room temperature comprising 20 gram colostrum and 1.2 gram NAC.

### Example 4. Nutritional composition for HIV patients as daily dose

| **Source material** | **g/day** | **Active component** | **Gram** |
|---|---|---|---|
| Protein | | | |
| Bovine colostrum powder | 16.9 | IgG (colostrum) | 4.0 |
| NAC | 1.8 | N-acetyl-L-cysteine | 1.8 |
| MPC | 1.5 | Milk protein | 1.5 |
| *Total Protein* | *20.2* | concentrate | |
| Fat blend | | | |
| Fish oil | 5.2 | EPA+DHA | 2.0 |
| Borage oil | 1.3 | GLA | 0.5 |
| *Total Fat* | 6.5 | | |
| Dietary Fiber/carbohydrates | | | |
| GOS syrup | 15.0 | Galactooligosaccharide | 6.75 |
| Inulin HP | 0.8 | Fructooligosaccharide | 0.75 |
| Pectin hydrolysate | 8.8 | Pectin | 7.5 |
| Fructose syrup | 17.5 | | |
| Glycerol | 4.5 | | |
| *Total fibers and CHO* | 46.6 | | |

## Claims

1. A nutritional composition particularly suited for HIV patients comprising colostrum and a fat blend comprising 10-40 wt% EPA, wherein colostrum is colostrum from cows.

2. The nutritional composition according to claim 1 wherein the fat blend further comprises between 1-25 wt% DHA (docosahexaenoic acid).

3. The nutritional composition according to claim 1 or 2 wherein the fat blend further comprises 1-15 wt% GLA (gamma linolenic acid).

4. The nutritional composition according to any of claims 1-3 wherein the n-3/n-6 fatty acid ratio of the fat blend is between 1-3 and the weight percentage of n-3 fatty acid is between 15-50%, and of n-6 fatty acid is between 10-50% of total fatty acid content of the fat blend.

5. The nutritional composition according to any of the claims 1-4 wherein the composition further comprises cysteine and/or N-acetyl cysteine (NAC), whey, egg proteins or a combination thereof providing at least 100 mg cysteine equivalent in a daily dose.

6. The nutritional composition according to any of the claims 1-5, said composition comprising between 15 and 50 en% lipid, between 25 and 65 en% colostrum protein, between 15 and 45 en% carbohydrate and cysteine or source of cysteine selected from the group consisting of NAC, whey, colostrum, egg proteins or combinations thereof.

7. Composition according to any of the claims 1-6 for use in the prevention and/or the treatment of intestinal dysfunction in HIV patients.

8. Composition for use in the prevention and/or the treatment of intestinal dysfunction in HIV patients according to claim 7 comprising colostrum protein between 5 and to gram in a daily dose.

9. Composition for use in the prevention and/or the treatment of intestinal dysfunction in HIV patients according to claim 7 or 8, wherein the nutritional composition comprises at least 0.15 g EPA in a daily dose.

10. Composition for use in the prevention and/or the treatment of intestinal dysfunction in HIV patients according to any of claims 7-9, wherein the nutritional composition comprises between 0.15 and 5 g EPA and between 0.05 and 2.5 g GLA in a daily dose.

11. Nutritional kit of parts comprising at least one solid composition a) and at least one liquid composition b), wherein compositions a) comprises colostrum from cows and preferably NAC, and composition b) comprises a fat blend comprising 10-40 wt% EPA, 1-25 wt% DHA and 1-15 wt% GLA.

## Patentansprüche

1. Eine insbesondere für HIV-Patienten geeignete Nahrungszusammensetzung umfassend Colostrum und eine Fettmischung umfassend 10 bis 40 Gew.-% EPA, worin das Colostrum Colostrum von Kühen ist.

2. Nahrungszusammensetzung nach Anspruch 1, worin die Fettmischung weiterhin zwischen 1 bis 25 Gew.-% DHA (Docosahexaensäure) enthält.

3. Nahrungszusammensetzung nach Anspruch 1 oder 2, worin die Fettmischung weiterhin 1 bis 15 Gew.-% GLA (Gamma-Linolensäure) enthält.

4. Nahrungszusammensetzung nach einem der Ansprüche 1 bis 3, worin das n-3/n-6-Fettsäure-Verhältnis der Fettmischung zwischen 1 bis 3 und der Gew.-Prozentsatz der n-3-Fettsäure zwischen 15 bis 50 % und der der n-6-Fettsäure zwischen 10 bis 50% des Gesamtfettsäuregehalts der Fettmischung liegt.

5. Nahrungszusammensetzung nach einem der Ansprüche 1 bis 4, worin die Zusammensetzung weiterhin Cystein und/oder N-Acetylcystein (NAC), Molke, Eiproteine oder Kombinationen davon umfasst, wodurch ein Äquivalent von mindestens 100 mg Cystein in einer Tagesdosis bereitgestellt wird.

6. Nahrungszusammensetzung nach einem der Ansprüche 1 bis 5, worin die Zusammensetzung zwischen 15 und 50 Energie-% (En.-%) Fett, zwischen 25 und 65 En.-% Colostrum-Protein, zwischen 15 bis 45 En.-% Kohlenhydrat und Cystein oder eine aus der Gruppe bestehend aus NAC, Molke, Colostrum, Eiproteinen oder Kombinationen davon ausgewählte Cystein-Quelle umfasst.

7. Zusammensetzung nach einem der Ansprüche 1 bis 6 zur Verwendung bei der Verhütung und/oder Behandlung intestinaler Dysfunktion bei HIV-Patienten.

8. Zusammensetzung zur Verwendung bei der Verhütung und/oder Behandlung intestinaler Dysfunktion bei HIV-Patienten nach Anspruch 7, umfassend zwischen 5 und 50 Gramm Colostrum-Protein in einer Tagesdosis.

9. Zusammensetzung zur Verwendung bei der Verhütung und/oder Behandlung intestinaler Dysfunktion bei HIV-Patienten nach Anspruch 7 oder 8, worin die Nahrungszusammensetzung mindestens 0,15 g EPA in einer Tagesdosis umfasst.

10. Zusammensetzung zur Verwendung bei der Verhütung und/oder Behandlung intestinaler Dysfunktion bei HIV-Patienten nach den Ansprüchen 7 bis 9, worin die Nahrungszusammensetzung zwischen 0,15 und 5 g EPA und zwischen 0,05 und 2,5 g GLA in einer Tagesdosis umfasst.

11. Nahrungs-Kit aus Teilen, umfassend mindestens eine feste Zusammensetzung a) und mindestens eine flüssige Zusammensetzung b), worin die Zusammensetzung a) Colostrum von Kühen und vorzugsweise NAC und Zusammensetzung b) eine Fettmischung, umfassend 10 bis 40 Gew.-% EPA, 1 bis 25 Gew.-% DHA und 1 bis 15 Gew.-% GLA umfasst.

## Revendications

1. Composition nutritionnelle particulièrement appropriée pour les patients atteints du VIH comprenant du colostrum et un mélange de matières grasses comprenant 10 à 40 % en poids d'EPA, le colostrum étant du colostrum issu de vaches.

2. Composition nutritionnelle selon la revendication 1, dans laquelle le mélange de matières grasses comprend en outre entre 1 et 25 % en poids de DHA (acide docosahexaénoïque).

3. Composition nutritionnelle selon la revendication 1 ou 2, dans laquelle le mélange de matières grasses comprend en outre 1 à 15 % en poids de GLA (acide gamma linolénique).

4. Composition nutritionnelle selon l'une quelconque des revendications 1 à 3, dans laquelle le rapport acide gras n-3/n-6 du mélange de matières grasses est entre 1 et 3 et le pourcentage en poids de l'acide gras n-3 est entre 15 et 50 %, et le pourcentage en poids de l'acide gras n-6 est entre 10 et 50 % de la teneur totale en acide gras du mélange de matières grasses.

5. Composition nutritionnelle selon l'une quelconque des revendications 1 à 4, dans laquelle la composition comprend en outre de la cystéine et/ou de la N-acétylcystéine (NAC), du lactosérum, des protéines d'oeuf ou une combinaison de ceux-ci, fournissant un équivalent d'au moins 100 mg de cystéine dans une dose journalière.

6. Composition nutritionnelle selon l'une quelconque des revendications 1 à 5, ladite composition comprenant entre 15 et 50 % en énergie de lipide, entre 25 et 65 % en énergie de protéine de colostrum, entre 15 et 45 % en énergie de glucide et de la cystéine ou une source de cystéine choisie dans le groupe constitué par de la NAC, du lactosérum, du colostrum, des protéines d'oeuf ou leurs combinaisons.

7. Composition nutritionnelle selon l'une quelconque des revendications 1 à 6, pour utilisation dans la prévention et/ou le traitement d'un dysfonctionnement intestinal chez les patients atteints du VIH.

8. Composition pour utilisation dans la prévention et/ou le traitement d'un dysfonctionnement intestinal chez les patients atteints du VIH selon la revendication 7, comprenant des protéines de colostrum entre 5 et 50 grammes dans une dose journalière.

9. Composition pour utilisation dans la prévention et/ou le traitement d'un dysfonctionnement intestinal chez les patients atteints du VIH selon la revendication 7 ou 8, dans laquelle la composition nutritionnelle comprend au moins 0,15 g d'EPA dans une dose journalière.

10. Composition pour utilisation dans la prévention et/ou le traitement d'un dysfonctionnement intestinal chez les patients atteints du VIH selon l'une quelconque des revendications 7 à 9, dans laquelle la composition nutritionnelle comprend entre 0,15 et 5 g d'EPA et entre 0,05 et 2,5 g de GLA dans une dose journalière.

11. Kit nutritionnel comprenant au moins une composition solide a) et au moins une composition liquide b), la composition a) comprenant du colostrum issu de vaches et de préférence de la NAC, et la composition b) comprenant un mélange de matières grasses comprenant 10 à 40 % en poids d'EPA, 1 à 25 % en poids de DHA et 1 à 15 % en poids de GLA.
